# EUROPEAN PATENT SPECIFICATION

(11) **EP 1 750 581 B1**
(45) Date of publication and mention of the grant of the patent: **12.02.2014**
(21) Application number: 05740582.1
(22) Date of filing: 10.05.2005
(51) Int. Cl.: A61B 5/08, G06F 19/00, G06N 3/02

(54) **MULTILEVEL VENTILATOR**
MEHRSTUFIGER VENTILATOR
RESPIRATEUR A PLUSIEURS NIVEAUX

(30) Priority: 10.05.2004 SE 0401208
(43) Date of publication of application: 14.02.2007
(73) Proprietor: Breas Medical AB, 435 33 Mölnlycke (SE)
(72) Inventor: HEDNER, Jan, S-412 66 Göteborg (SE); KNAGENHJELM, Petter, S-435 43 Pixbo (SE); TIEDJE, Mikael, S-425 30 Hisings Backa (SE); GROTE, Lutger, S-412 68 Gothenburg (SE)
(74) Representative: Valea AB
(86) International application number: PCT/SE2005/000679
(87) International publication number: WO 2005/107589

(56) References cited:
- EP-A1- 1 371 384
- WO-A1-02/28281
- US-A- 5 765 563
- US-A- 5 953 713
- VARADY P. ET AL: 'A Novel Method for the Detection of Apnea and Hypopnea Events in Respiration Signals' IEEE TRANSACTIONS ON BIOMEDICAL ENGINEERING vol. 49, no. 9, September 2002, pages 936 - 942, XP002991642

## Description

### Field of the invention

The present invention relates to the measurement and control of breathing gas administration into humans, and more specifically automatic, adaptive and learning control mechanisms for detection and treatment of breathing disorders. The learning control system uses one or several artificial neural network algorithms with several measured input parameters and a database obtained from a large number of prior measurements on a large group of subjects to calculate an appropriate response to a detected breathing disturbance.

### Background of the invention

Patients suffering from different forms of breathing disorders can be subject to several types of treatments depending on the illness or disorder present. Such treatments include surgical procedures, pharmacologic therapy, and non-invasive mechanical techniques. Surgical techniques to remedy breathing disorders constitute a considerable risk for the patient and can lead to permanent injury or even mortality. Pharmacologic therapy has in general proved disappointing with respect to treating certain breathing disorders, e.g. sleep apnea. It is therefore of interest to find other treatments, preferably non-invasive techniques.

A mechanical ventilator represents a non-invasive technique for treatment of certain breathing disorders such as ventilatory failure, hypoventilation, and periodic breathing during sleep and awake and in sleep apnea that occurs exclusively during sleep. Ventilatory failure includes all forms of insufficient ventilation with respect to metabolic need whether occurring during wake or periods of sleep. Hypoventilation and periodic breathing, in its most frequently occurring form referred to as Cheyne-Stokes ventilation, may occur periodically or constantly during wake or sleep. Conditions associated with hypoventilation, in particular nocturnal hypoventilation include e.g. central nervous system disorders such as stroke, muscular dystrophies, certain congenital conditions, advanced chronic obstructive pulmonary disease (COPD), etc. Cheyne-Stokes ventilation or various forms of central apnea are commonly associated with cardiac and circulatory disorders, in particular cardiac failure.

Sleep apnea can be categorized into two different forms that occur selectively or in combination. In central sleep apnea, a primarily central nervous system coordination disorder, all respiratory movement is interrupted leading to a sleep apnea event. Obstructive sleep apnea, in contrast, is associated with upper airway collapse, presumably caused by loss of or inadequate upper airway muscle tone. This condition is particularly likely to occur in subjects with narrow upper airways due to excess soft tissue or anatomical abnormalities. Obstructive sleep apnea is the most common type of sleep apnea event. Apnea events are considered pathological, for instance, if they exceed 10 seconds in duration and if they occur more frequently than 10 times per hour of sleep.

Ventilatory failure is a potentially life threatening condition. The general comorbidity in patients with failing ventilation is considerable. The condition is highly disabling in terms of reduced physical capacity, cognitive dysfunction in severe cases and poor quality of life. Patients with ventilatory failure therefore experience significant daytime symptoms but in addition, the majority of these cases experience a general worsening of their condition during state changes such as sleep. The phenomenon of disordered breathing during sleep, whether occurring as a consequence of ventilatory failure or as a component of sleep apnea in accordance with the description above causes sleep fragmentation. Daytime complications include sleepiness and cognitive dysfunction. Severe sleep disordered breathing occurring in other comorbid conditions like obesity, neuromuscular disease, post polio myelitis states, scoliosis or heart failure may be associated with considerable worsening of hypoventilation and compromised blood gas balance. Sleep apnea has been associated with cardiovascular complications including coronary heart disease, myocardial infarction, stroke, arterial hypertension, thrombosis, and cardiac arrhythmia. It is therefore of both immediate and long-term interest to reduce the exposure to sleep disordered breathing.

Recent advancement in mechanical non-invasive ventilator techniques includes administration of continuous positive airway pressure (CPAP) in different forms of sleep disordered breathing. During CPAP administration an elevated airway pressure is maintained throughout the breathing phase during a period coinciding with sleep. In sleep apnea this procedure may provide appropriate stabilization of the upper airway thereby preventing collapse. This, so called mono-level CPAP therapy, provides an almost identical pressure during inhalation and exhalation. Not only may CPAP be uncomfortable for the patient due to a sensed increased work of breathing during ventilation, specifically expiration. Some forms of apnea, mainly including those of central origin, and most forms of hypoventilation are only poorly controlled by CPAP. A more recently developed bi-level CPAP system administers different pressure levels during inhalation and exhalation. Bi-level CPAP provides increased comfort for most patients and not infrequently, an improved clinical response. Bi-level CPAP provides two pressure levels, Inspiratory Positive Airway Pressure (IPAP) and Expiratory Positive Airway Pressure (EPAP). IPAP is administered during the inhalation phase while EPAP is given during the exhalation phase.

Clinically applicable IPAP and EPAP levels typically need to be identified, which may be done during a sleep study conducted in a sleep laboratory.

One such bi-level CPAP system can be found in US 6,629,527 which disclose a system for detecting the presence or absence of a patient in the breathing air interface and which automatically turns on or off the CPAP treatment accordingly. The system also has a ramp system that controls the pressure levels and changes them over a period of time in order to allow the patient to fall asleep. This ramp cycle may for example, be activated by the user through a remote control.

However, during the course of the sleeping period the physiological pressure or air flow need constantly changes in association with alterations of sleep stages and factors such as e.g. sleep position. A standard bi-level CPAP system can cause discomfort during sleep and may even arouse the user due to the static configuration of the standard bi-level CPAP system. This is of course highly undesirable and thus there is a need for methods to automatically adjust the mechanical ventilator settings in order to better reflect the need and sleeping mode of the patient.

US patent 5,953,713 discloses a method for treating sleep disordered breathing comprising a measurement of the pressure at the ventilator interface placed over the airway of the patient. The measured data is processed in a Fast Fourier Transform (FFT) and the obtained frequency data is fed into a neural network trained to recognize patterns characterizing sleep disordered breathing. Since this system uses a frequency analysis, it has to find a balance between sampling frequency and number of sampling data. This may unfortunately affect the response time and lead to a slower response time, thus creating a risk of a progressive deterioration of the airway aperture.

WO 02/28284 discloses a method and apparatus for the detection and treatment of disordered breathing during sleep using an artificial neural network operating on a measured breathing gas flow parameter of the pressurized breathing gas controlled by CPAP. The artificial neural network is trained with data obtained from a large population of patients and data obtained from a sleep study of the specific patient in a sleep laboratory. The solution presented in WO 02/28284, only configures the overall CPAP pressure, but this is a limitation since other parameter changes may be beneficiary for the patient. This document describes an example of an automated CPAP system where the CPAP system identifies and treats obstructed breathing utilizing an advanced processing technique in a self-adjusting device and used in a product manufactured by BREAS AB under the product name of PV10i.

### Summary of the invention

It is an object of the present invention to provide a system that remedies the above mentioned problems and provides an improved breathing gas ventilator technology for use in conjunction with ventilatory disorders, such as for instance as a continuous positive airway pressure (CPAP) method, such as an automatic adaptive CPAP (AACPAP).

Using a sensor system measuring one or several input parameters related to a subjects breathing pattern alone or in combination with other physiological variables, exemplified but not limited to for instance, airflow and airway or mask system pressure , snoring events, heart rate, muscle tone or activity derived by actugraphy or electromyography or the like, electroencephalographic signals, skin circulation, blood pressure and so on, and using these data in an artificial neural network processing system it is possible to better control the breathing support in order to increase its relative effectiveness and/or to provide increased therapy comfort for the user. The method controls several different parameters governing the pressure and flow of breathing air support used in a CPAP system. For instance the ratio between an EPAP and IPAP pressure (EPAP = Expiratory Positive Airway Pressure, IPAP = Inspiratory Positive Airway Pressure), as well as the absolute flow levels can be controlled.

An example of an artificial neural network that can be used in this analysis is described in detail in for instance WO 02/28281. However, it should be understood that the present invention is not limited to this artificial neural network scheme but it should be appreciated that other algorithms may be used.

In the present invention the artificial neural network consists of one or a plurality of artificial neural network algorithms. These can be the same or different algorithms. Each artificial neural network or networks acts on one or several measured input parameters that reflect the physiological state of the patient, for instance one or more of the following signals; gas flow, gas pressure, electromyography (EMG) signals, electroencephalogram (EEG) signals, electrocardiogram (ECG) signals, blood pressure, heart rate, eye movements for instance from electrooculograms (EOG), and sound events indicative of a breathing disorder, such as snoring or similar.

The artificial neural network operates on sampled data from sensor signals relating to the physiological status of a patient and it may specifically operate on flow/pressure data which has been divided into intervals either related to a predefined time factor or intervals indicative of different phases of the breathing duty cycle.

The neural network controls and decides on an appropriate response for setting the flow and/or pressure curve form or ramp cycles in order to keep a comfortable breathing pattern setting for the patient. In for instance a CPAP system, the artificial neural network can control for instance the upper and lower levels of the flow and/or pressure response. The upper level is used during the inhalation part of the breathing process and thus called IPAP (inspiratory positive airway pressure). The lower level is used during the exhalation part of the breathing process and thus called EPAP (expiratory positive airway pressure).

Several artificial neural networks can act on the same input parameter but be used for determining different control signal parameters.

The invention is defined in the claims.

### Brief description of the drawings

Fig. 1 is a schematic depiction of a ventilatory system according to the present invention.
Fig. 2 shows a schematic diagram over one embodiment of an artificial neural network system according to the present invention.
Fig. 3 shows a schematic diagram over another embodiment of an artificial neural network system according to the present invention.
Fig. 4 shows a diagram of an exemplifying pressure and flow signal versus time according to the present invention.
Fig. 5 shows a schematic example of a ramp cycle test pattern for obtaining a relevant EPAP and IPAP ratio and level used in the treatment of a breathing disorder.
Fig. 6 is a schematic illustrating an exemplary method.

### Detailed description of the invention

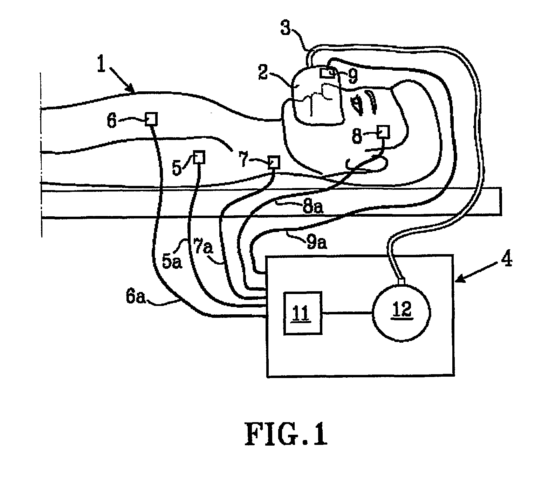

In Fig. 1 a schematic mechanical ventilation system used for the treatment of hypoventilation disorders is depicted. A ventilation system comprise a mechanical ventilator 4 supplying pressurized breathing gas, tubing 3 for guiding breathing gas to the patient 1, a breathing mask 2 or similar for administrating the breathing gas to the patient 1, sensing means 5, 6, 7, 8, and 9 for determining the physiological status of the patient 1. A mechanical ventilator 4 is supplying breathing gas for instance as a positive airway pressure via a tubing 3 and through a mask 2 to a patient 1. The mask 2 can be a face mask 2 covering both the mouth and nose or a nasal mask covering only the nose or nostrils depending on the patients needs. It can also be a hood covering the complete head or body of the patient.

The breathing gas may be of any suitable gas composition for breathing purposes as understood by the person skilled in the art, the composition depending on the physiological status of the patient.

The pressure or flow from the ventilator 4 is controlled by a processing unit 11 as shown in Fig. 1. The processing unit 11 measures one or several input parameters 5, 6, 7, 8, and 9 obtained from the patient 1 describing the physiological status of the patient. Data indicative of patient status is obtained using sensors 5, 6, 7, 8, and 9 connected to the patient and transferred to the processing unit 11 via connection means 5a, 6a, 7a, 8a, and 9a. These input parameters may be for instance flow or pressure signals, data obtained from EEG, EMG, EOG, and ECG measurements, O₂ and/or CO₂ measurements in relation to the patient, body temperature, blood pressure, SpO2 (oxygen saturation), eye movements, and sound measurements. It should be understood that the invention is not limited to the above mentioned input parameters but other input parameters may be used. In Fig. 1 not all sensors 5, 6, 7, 8, and 9 and sensor connection means 5a, 6a, 7a, 8a, and 9a are depicted, only a subset is shown in order to illustrate a schematical view of the system and the depicted locations are only given as examples and are in no way limiting to the invention.

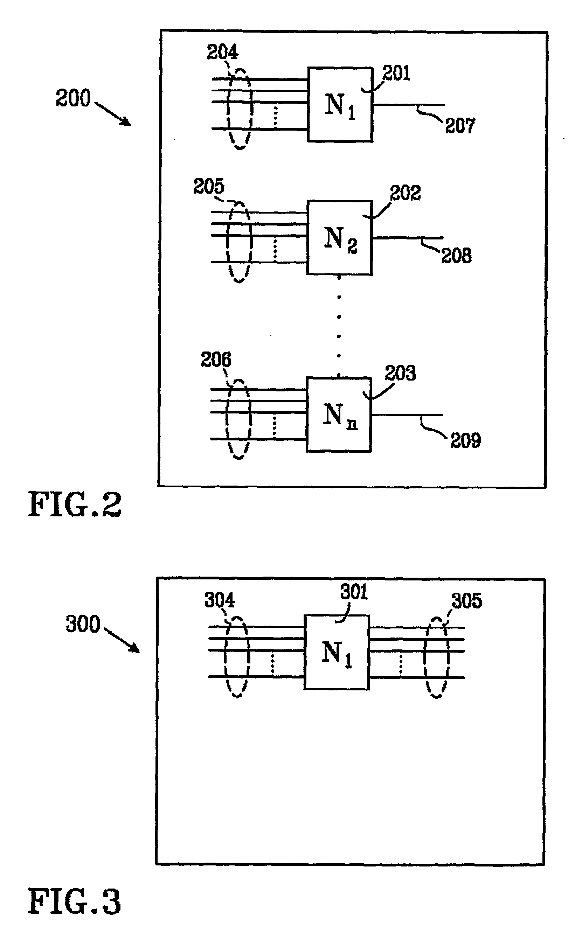

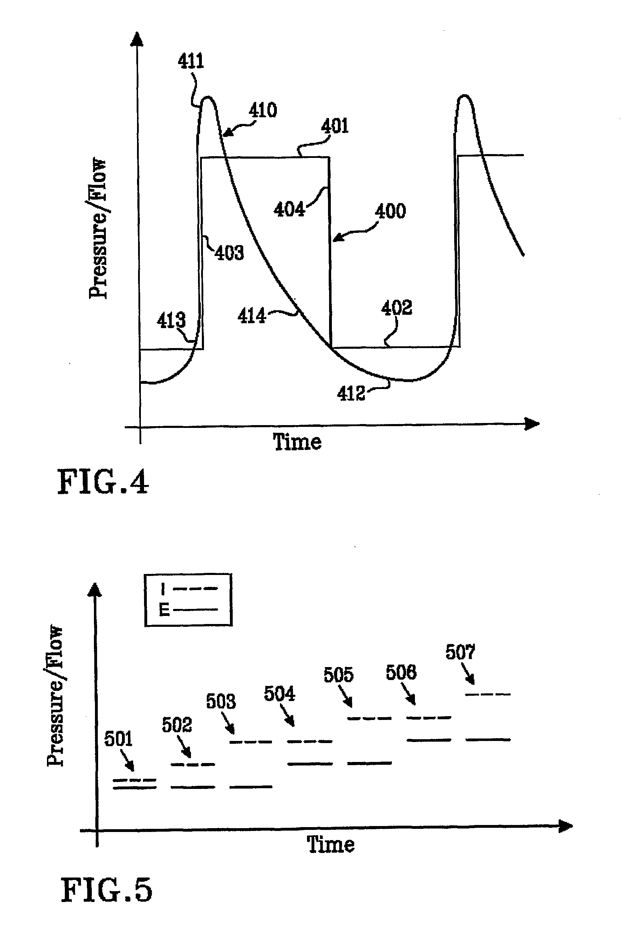

The input data is supplied to a processing unit 11 comprising of computational means and communicative means and at least one artificial neural network algorithm acting on one or several of the inputted data. The neural network system may consist of one or several independent or dependent artificial networks deducing control settings for a ventilator apparatus 4. In one preferred embodiment, several artificial neural networks 201, 202, up to 203 each deduce one or several parameter settings 207, 208, up to 209 for instance as seen in Fig. 2 where N1 201 is one network and N2 202 is another network and so on for Nn networks 203. In the example of Fig. 2, network N1 201 has the responsibility to deduce a single control parameter 207 controlling for instance the IPAP level 401 as seen in Fig. 4 and network N2 202 has the responsibility to deduce a control parameter 208 for controlling for instance the EPAP level 402. Other vital parameters that may be calculated are for instance duration time of the breathing duty cycles or frequency of breathing duty cycles.

In the mechanical ventilator device 4 there is also a data storage unit for post analysis and inspection and there can also be a connection for an external nonvolatile memory device, like for instance a memory device using a USB connection, an external hard drive, a floppy disk, a CD-ROM writer, a DVD writer, a Memorystick, a Compact Flash memory, a Secure Digital memory, an xD-Picture memory card, or a Smart Media memory card. These are only given as examples, and are not limiting for the invention, many more external memory devices may be used in the invention.

The mechanical ventilator also has input means (not shown) for manually setting control parameters and other parameters necessary for the operation of the device.

Through a communication means (not shown) it is possible to communicate with the device 4 to and from an external computational device for retrieving data and results for later analysis and/or inspection. The communication means can be of a serial type like for instance according to the standards RS232, RS485, USB, Ethernet, or Firewire, or of a parallel type like for instance according to the standards Centronics, ISA, PCI, or GPIB/HPIB. It may also be any wireless system of the standards in the IEEE 802.11 series, HiperLAN, Bluetooth, IR, GSM, GPRS, or UMTS, or any other appropriate fixed or wireless communication system. It can also be of any proprietary non-standardized communication formats, whether it is wireless or wired.

The ventilator device 4 may also have display means (not shown) for displaying measured data and obtained response parameters for use by a physician, other medical personnel, or the patient. The display means may be of any normal type as appreciated by a person skilled in the art. The data is displayed with such a high rate that a real time feedback is provided to a person monitoring the ventilator characteristics and function for immediate feedback and control.

In the present invention several artificial neural networks may be used in order to determine hypoventilation events and periods, determining an appropriate response, and controlling a ventilator device 4 accordingly to the determined response. The system acquires pressure/flow curves using the sensors 5, 6, 7, 8, and 9 and divides the acquired curves into segments according to either predefined time factor settings (such as fixed time lengths, e.g. a number of seconds) or preferably into segments indicative of breathing cycle phase as understood by the person skilled in the art. These breathing cycle phases are determined by large changes in the flow/pressure curve. Looking at Fig. 4 and the pressure signal 400, the different breathing cycle phases may be divided for example into four segments, illustrated in the following exemplifying phases:
1. the inspiratory phase 401 when the patient is inhaling breathing gas (IPAP level, Inspiratory Positive Airway Pressure);
2. the transfer phase 404 between the inspiratory phase 401 and the expiratory phase 402;
3. the expiratory phase 402 when the patient exhales the breathing gas (EPAP level, Expiratory Positive Airway Pressure); and
4. the transfer phase 403 from the expiratory phase 402 to the inspiratory phase 401.

Each breathing phase can provide information about different types of breathing disorders, since these disorders will affect the breathing phases differently. This may be used in an analysis of the breathing phases, by analyzing the different breathing phases separately and thus provide indications of different breathing disturbances and subsequently used in different analysis schemes in order to determine the patient status and for determining appropriate responses and ventilator control parameters according to determined status.

Each artificial neural network in the artificial neural network system is responsible for determining a control parameter: one artificial neural network is used to determine the IPAP level, another artificial neural network is used to determine the EPAP level, yet another artificial neural network is used to determine an airway pressure level duration time, one artificial neural network is used to determine a frequency of applied airway pressure breathing cycle, one artificial neural network is used to determine a pressure change cycle for use in determining appropriate pressure settings and initiation of said pressure change cycle, and one artificial neural network is used to determine a pressure change cycle for use in response of a determined status of the patient and initiation of said pressure change cycle.

The processing unit may also be used to determine change cycles in order to iteratively acquire an appropriate configuration of EPAP 402 and IPAP 401 levels and ratios. Such an iterative process can be seen in Fig. 5, where it should be understood that the illustrated levels and sequence of iteration is only for illustrative purposes and not limited to these values, directions and order. The starting point may for instance be with both the IPAP and EPAP values at the same level 501. The system then raises the IPAP level but keeps the EPAP level constant 502. After this the system measures the response from the patient and if the configuration is still not satisfactorily the IPAP level is raised again 503. A new patient response measurement is done and another adjustment is done 504. This process is continued with subsequent adjustments 505, 506, 507, and so on until the response from the patient is deemed satisfactory. There is a maximum ratio between the IPAP and EPAP values that is allowed and this ratio is deduced from the patient specific criteria, for instance from reference measurement of sleep apnea events in a sleep laboratory or from other measurable patient characteristics exemplified by weight, height, airway aperture, neck circumference, type and severity of the breathing disorder etc.

Such a pressure change cycle can also be used for adjusting to slow changes in the patient breathing pattern consistent with physiological changes, like for instance when the patient fall a sleep and slowly lowers the breathing rate and volume, again Fig. 5 may be used for illustrating this embodiment. The control system initiates a pressure change cycle upon the diagnosis or detection of such an event or physiological change. Other examples of this type of usage of pressure change cycles may be utilized as would be appreciated by the person skilled in the art.

In another embodiment a single artificial neural network 301 is used in the artificial neural network system 300 for the deduction of several control parameters 305 with a plurality of input parameters 304. Otherwise the control and possible treatment methods are the same as for the above mentioned artificial neural network using a plurality of networks 201, 202, and 203. This single neural network 301 also inputs a plurality of measured sensor signals 304 either directly or indirectly through some kind of pre-processing means. The neural network 301 deduces one or several control parameters 305 for controlling the breathing gas ventilation system 4.

In another preferred embodiment (not shown) a combination of the above two described embodiments may be utilized: several artificial neural networks 201, 202, and 203 are used and each artificial neural network deduce several control parameters. For instance one artificial neural network may be dedicated to suggesting appropriate IPAP and EPAP levels after determining a specific patient status, another network is dedicated to suggesting duty cycles in a similar fashion, and yet another network is dedicated to determining patient status resulting in specific cycle pattern changes.

The above described method and apparatus may be used to determine many different control parameters concerning a breathing gas ventilation apparatus or method including but not limited to:
1. IPAP level
2. EPAP level
3. breathing duty cycles, including cycle rate (frequency) and time duration of different parts of the breathing duty cycle.
4. pressure limit
5. work of breathing (WOB)
6. pressure support ventilation (PSV) level
7. tidal volume (V_{T}) level
8. continuous positive airway pressure (CPAP)
9. positive end expiratory pressure (PEEP)
10. fractional inhaled oxygen concentration (FiO2) level
11. breathing gas flow rate level
12. minute ventilation level

The tidal volume (V_{T}) level is the volume of breathing gas moving in and out of the lungs per breath. PEEP is a baseline of elevated positive pressure maintained during inhalation and exhalation during machine assisted ventilation. FiO2 level is the amount of oxygen in the air inhaled. Minute ventilation level is the volume of breathing gas moving in and out of the lungs per minute.

Before an artificial neural network can be used it has to be trained using previously acquired data. These are typically obtained from continuous recordings performed in previous patients or from control groups, as well as from descriptive data on typical patient characteristics. These data are fed to the artificial neural network in a fashion earlier described in for instance WO 02/28281. The neural network uses also data obtained for the specific patient during a reference measurement, for example, in relation to breathing disturbances during sleep, limited channel recording during sleep is often done in a sleep laboratory during one or more sessions. The screening process is performed to observe and measure a specific patient responses to a breathing disturbance, for instance for sleep apnea events, and to find basic settings and safety levels for a ventilator apparatus, e.g. a CPAP apparatus. These data are later combined into criteria or templates for the neural network system to be used in the calculational process for a specific patient.

Some different examples of usage and situations concerning hypoventilation events during sleep will now be given according to the present invention in relation to breathing disturbances during sleep. It should be understood that the below mentioned examples is only made for illustrative purposes and is not made as a limitation to the present invention.
1. Based on the above described method and apparatus, measurements done for the detection of flow limitations, obstructions, snoring, and apneas, the system will alter the ratio between IPAP and EPAP pressures using a predetermined criterion or template in the artificial neural network. If an apneic period is detected the ventilator generates backup breaths at a calculated rate based on the predetermined criterion/criteria.
2. Using two artificial neural networks with input from flow and pressure measurements the IPAP and EPAP pressures are controlled separately for maximum comfort for the patient. If the patient suffers from a sleep apnea event the system will provide a backup breath frequency either calculated from preceding breaths or given as the set backup frequency.
3. A predetermined pattern in EPAP and IPAP pressure profiles can be applied using an artificial neural network. The profile can be deduced from a large number of measurements from previous patients and/or reference groups. For instance upon the detection of a flow restriction during en EPAP period the EPAP could raised with 1 cmH₂O during 1 min along with an increase of IPAP pressure, or if an apnea period is detected the EPAP and IPAP ratio can be increased during 3 min. to increase ventilation. This type of response is illustrated in Fig. 5. The above-mentioned pressure and time parameters are only given as examples and should not be interpreted as a limitation to the invention.

Depending on the chosen scheme and input parameters different pre-processing procedures will be utilized in order to feed the artificial neural network with appropriate data and in an appropriate data format, for instance, digitizing the data, scaling data to a predefined level, normalizing data, vectorization, or structuring the data in an appropriate format, and so on.

The artificial neural network will, for a given measured data sequence, determine the most likely hypoventilation event from the database, described in WO 02/28281 for the specific patient by comparing the input parameters/data with the templates in the database. This is done on a subset of data from the sensor or sensors input stream/streams. As an example, the determined event can be a flow limitation. The neural network continues to acquire data and calculate and then determines the next event by working on a subset of the data offset by a small distance wherein part of the former subset is also present. For examples sake, again the determined event might be a flow limitation. If the calculations for a certain number of times determine that a flow limitation is present for the patient the control system triggers a response appropriate for a flow limitation. This can be done for many types of hypoventilation events, for example, but not limited to, flow limitations, flow apnea both for central apnea and obstructive apnea or a combination of these two apnea types, and other hypoventilation events. In such a way may the patient breathing pattern be analyzed in real-time during a treatment process, the type of hypoventilation is determined, all data is stored in the memory of the device 4, and appropriate action is taken considering the administration of pressure.

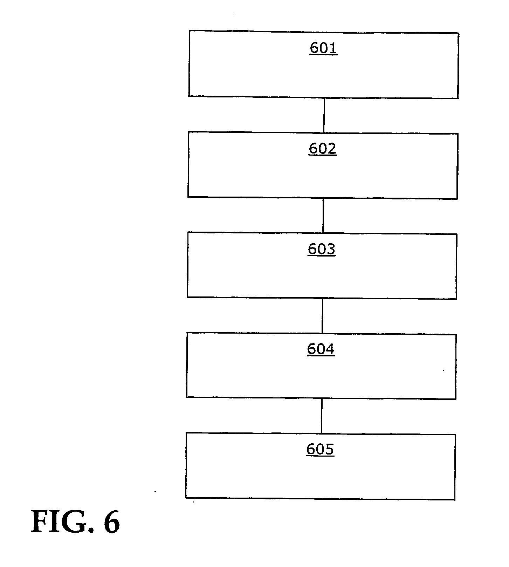

A method for using the above mentioned apparatus is illustrated in Fig. 6. The method detects hypoventilation events and controls breathing assistance patterns in a breathing gas ventilator system using an artificial neural network system 200, 304. The method comprise the following steps:
- Periodically acquiring 601 samplesd input parameters 5, 6, 7, 8, and 9 indicative of patient physiological status.
- Dividing 602 a flow or pressure curve obtained from the sampled input parameters 5, 6, 7, 8, and 9, into two or more intervals using a predefined time factor or intervals indicative of patient 1 breathing cycle phases.
- Finding 603 a hypoventilation event directly or indirectly from the input parameters and/or the divided flow or pressure curve using the artifical neural network system 200, 300.
- Determining 604 an appropriate response from predetermined criteria for a breathing gas ventilator apparatus 4 using the above mentioned finding 603 result.
- Generating 605 output control signals 207, 208, 209, 305 indicative of control signals for a mechanical ventilator apparatus 4 for controlling the ventilator apparatus 4 according to the determined response.

There are many types of ventilation modes where the above described method and apparatus may find its application including but not limited to:
1. Continuous positive airway pressure (CPAP),
2. Synchronized intermittent mandatory ventilation (SIMV),
3. Assist control mechanical ventilation (ACMV),
4. Pressure control ventilation (PCV),
5. Pressure support ventilation (PSV),
6. Proportional assist ventilation (PAV), and
7. Volume assured pressure support (VAPS)

These kinds of methods and devices are often used for treating disturbed breathing during for instance sleep either in the home or in a clinical environment. The methods and devices described above within the scope of the invention may also be used for treatment of many other different forms of ventilatory failure events or hypoventilation events, and treatment may be done both at home and in the clinical environment. Examples of groups of breathing disorders include, but are not limited to, breathing disorders during sleep, obstructive lung diseases (COPD), neuromuscular disorders, neurological disorders, chest wall disorders, and more.

The above mentioned and described embodiments are only given as examples and should not be limiting to the present invention. Other solutions, uses, objectives, and functions within the scope of the invention as claimed in the below described patent claims should be apparent for the person skilled in the art.

### Reference signs

- 1: Patient
- 2: Mask
- 3: Tubing
- 4: Screening device
- 5: Sensor
- 5a: Connection means
- 6: Sensor
- 6a: Connection means
- 7: Sensor
- 7a: Connection means
- 8: Sensor
- 8a: Connection means
- 9: Sensor
- 9a: Connection means
- 11: Processing unit
- 12: Pressure/flow regulator
- 200: Artificial neural network system
- 201: Artificial neural network N1
- 202: Artificial neural network N2
- 203: Artificial neural network Nn
- 204: Input parameter or parameters
- 205: Input parameter or parameters
- 206: Input parameter or parameters
- 207: Output parameter
- 208: Output parameter
- 209: Output parameter
- 300: Artificial neural network system
- 301: Artificial neural network
- 304: Input parameter or parameters
- 305: Output parameter or parameters
- 400: Pressure signal
- 401: IPAP level of pressure signal
- 402: EPAP level of pressure signal
- 403: Rise slope of the pressure
- 404: Fall slope of the pressure
- 410: Flow signal
- 411: "IPAP" level of the flow
- 412: "EPAP" level of the flow
- 413: Rise slope of the flow
- 414: Fall slope of the flow
- 501-507: IPAP (I) and EPAP (E) level settings at a different time instants

## Claims

1. An apparatus for the detection of hypoventilation events and for controlling breathing assistance patterns employlng an artificial neural network system, the apparatus comprises one or several sensor input channels (5, 6, 7, 8, 9) for periodic sampling of data consistent with a patients physiological status, in particular related to breathing status, computational means (11) comprising an artificial neural network system (200, 304), for direct or indirect analysis of said data, for determining control parameters for control of breathing gas pressure in a breathing assisting apparatus (4), **characterized in that** said sampled data is related to a flow or pressure curve being divided into two or more intervals (401, 402, 403, 404) indicative of patient (1) breathing cycle phases prior to being fed to said artificial neural network (201, 202, 203, 301).

2. The apparatus according to claim 1, **characterized in that** said artificial neural network system (200) comprises one or several independent artificial neural networks (201, 202, 203) each determining different response parameters (207, 208, 209).

3. The apparatus according to claim 2, **characterized in that** said determined response parameters (207, 208, 209, 305) comprise one or several of inspiratory positive airway pressure level (401), IPAP, expiratory positive airway pressure level (402), EPAP, pressure limit, pressure support ventilation (PSV) level, tidal volume (V_{T}) level, continuous positive airway pressure (CPAP), positive end expiratory pressure (PEEP), fractional inhaled oxygen concentration (FiO2) level, breathing gas flow rate level, and minute ventilation level.

4. The apparatus according to claim 2, **characterized in that** said periodically sampled input parameters (5, 6, 7, 8, 9) comprise signals indicative of one or several of gas flow, gas pressure in airway or mask system, muscle tone derived by actugraphy or electromyography (EMG) signals, electroencephalagram (EEG) signals, electrooculargrams (EOG), electrocardiogram (ECG) signals, blood pressure, O₂ level, CO₂ level, SpO2 (oxygen saturation) level, eye movement, skin circulation, and sound events indicative of a breathing disorder.

## Patentansprüche

1. Vorrichtung zur Detektion von Hypoventilations-Vorfällen und zur Steuerung von Beatmungsassistenzmustern mittels eines künstlichen neuronalen Netzwerk-Systems, wobei die Vorrichtung einen oder mehrere Sensor-Eingangs-Kanäle (5, 6, 7, 8, 9) zur periodischen Abtastung von Daten aufweist, die mit dem physiologischen Zustand eines Patienten, insbesondere bezogen auf den Zustand seiner Atmung, konsistent sind, Rechenmittel (11), ein künstliches neuronales Netzwerk-System (200, 304) aufweisend, zur direkten oder indirekten Analyse der Daten, um Steuerparameter zur Steuerung des Beatmungsgasrucks in einer Beatmungsassistenzvorrichtung (4) zu bestimmen,
**dadurch gekennzeichnet, dass**
die abgetasteten Daten mit einer Fluss- oder Druck-Kennlinie in Bezug gesetzt werden, die in zwei oder mehr Intervalle (401, 402, 403, 404) aufgeteilt ist, welche Atmungszyklusphasen des Patienten (1) aufzeigen, bevor sie dem künstlichen neuronalen Netzwerk (201, 202, 203, 301) zugeführt werden.

2. Vorrichtung gemäß Anspruch 1, **dadurch gekennzeichnet, dass** das künstliche neuronale Netzwerk-System (200) ein oder mehrere künstliche neuronale Netzwerke (201, 202, 203) aufweist, wobei jedes unterschiedliche Antwortgrößen (207, 208, 209) bestimmt.

3. Vorrichtung gemäß Anspruch 2, **dadurch gekennzeichnet, dass** die bestimmten Antwortgrößen (207, 208, 209) eines oder mehrere der inspiratorischen positiven Atemwegsdruck-Niveaus (401), IPAP, exspiratorischen positiven Atemwegsdruck-Niveaus (402), EPAP, Druckgrenzen, Druckunterstützungs-Niveaus (PSV), Atemzugvolumen (Tidalvolumen, V_{T}), kontinuierlichen positiven Atemwegsdrucks (CPAP), positiven und exspiratorischen Drucks (PEEP), Niveau der fraktionalen eingeatmeten Sauerstoff-Konzentration (FiO2), Niveau des Atemgas-Flussrate und Menge geatmeter Luft pro Minute aufweisen.

4. Vorrichtung gemäß Anspruch 2, **dadurch gekennzeichnet, dass** die periodisch abgetasteten Eingangs-Parameter (5, 6, 7, 8, 9) Signale aufweisen, die einen oder mehrere der Gas-Fluss, Gasdruck im Atemweg- oder Masken-System, Muskel-Tonus, der aus Aktugraphie- oder Elektromyographie-Signalen (EMG) abgeleitet wurde, Elektroenzephalogramm-Signale (EEG), Electrookularogramme (EOG), Elektrokardiogramm-Signale (EKG), Blutdruck, O₂ Niveau, CO₂ Niveau, SpO₂-Niveau (SauerstoffSättigung), Augenbewegung, Durchblutung der Haut und akustische Ereignisse (sound events), die auf Atmungsstörungen hinweisen, anzeigen.

## Revendications

1. Appareil de détection d'événements d'hypoventilation et de régulation de profils d'assistance respiratoire à l'aide d'un système de réseau neuronal artificiel, l'appareil comprenant un ou plusieurs canaux d'entrée de capteur (5, 6, 7, 8, 9) pour l'échantillonnage périodique de données en rapport avec un statut physiologique d'un patient, en particulier relatives à l'état respiratoire, un moyen de calcul (11) comprenant un système de réseau neuronal artificiel (200, 304), pour l'analyse directe ou indirecte desdites données, pour déterminer les paramètres de régulation pour réguler la pression de gaz respiratoire dans un appareil d'assistance respiratoire (4), **caractérisé en ce que** lesdites données échantillonnées sont liées à une courbe de débit ou de pression divisée en deux intervalles ou plus (401, 402, 403, 404) indiquant les phases du cycle respiratoire d'un patient (1) avant d'être envoyées audit réseau neuronal artificiel (201, 202, 203, 301).

2. Appareil selon la revendication 1, **caractérisé en ce que** ledit système de réseau neuronal artificiel (200) comprend un ou plusieurs réseaux neuronaux artificiels indépendants (201, 202, 203) déterminant chacun différents paramètres de réponse (207, 208, 209).

3. Appareil selon la revendication 2, **caractérisé en ce que** lesdits paramètres de réponse prédéterminés (207, 208, 209, 305) comprennent un ou plusieurs du niveau de pression inspiratoire positive (401), IPAP, du niveau de pression expiratoire positive (402), EPAP, de la limite de pression, du niveau de ventilation de support de pression (PSV), du niveau de volume courant (V_{T}), de la pression respiratoire positive continue (CPAP), de la pression positive en fin d'expiration (PEEP), du niveau de concentration fractionnelle de l'oxygène inspiré (FiO₂), du niveau de débit du gaz respiratoire, et du niveau de ventilation minute.

4. Appareil selon la revendication 2, **caractérisé en ce que** lesdits paramètres d'entrée périodiquement échantillonnés (5, 6, 7, 8, 9) comprennent des signaux indiquant un ou plusieurs du flux de gaz, de la pression gazeuse dans le système respiratoire ou de masque, du tonus musculaire obtenu par des signaux actimétriques ou électromyographiques (EMG), des signaux électroencéphalographiques (EEG), d'électro-oculogrammes (EOG), des signaux électrocardiographiques (ECG), de la pression sanguine, du niveau d'O₂, du niveau de CO₂, du niveau de SpO₂ (saturation en oxygène), du mouvement oculaire, de la circulation cutanée et des événements sonores indiquant un trouble respiratoire.
